# EUROPEAN PATENT APPLICATION

(11) **EP 4 439 445 A1**
(43) Date of publication of application: **02.10.2024**
(21) Application number: 23305444.4
(22) Date of filing: 30.03.2023
(51) Int. Cl.: G06T 5/50, G06V 20/00

(54) **DEVICE, SYSTEM AND METHOD FOR GENERATING A MEDICAL IMAGE OF A REGION OF INTEREST OF A SUBJECT INDICATING CONTRAST-ENHANCED REGIONS**

(71) Applicant: Koninklijke Philips N.V., 5656 AG Eindhoven (NL)
(72) Inventor: WIEMKER, Rafael, 5656 EINDHOVEN (NL); BONTUS, Claas, 5656 EINDHOVEN (NL); MATUTE FLORES, Jose Alejandro, 5656 EINDHOVEN (NL); GROTH, Alexandra, 5656 EINDHOVEN (NL); HEESE, Harald, 5656 EINDHOVEN (NL); NICKISCH, Hannes, 5656 EINDHOVEN (NL); PETERS, Jochen, 5656 EINDHOVEN (NL); VLACHOMITROU, Anna Sesilia, 5656 EINDHOVEN (NL); WEESE, Juergen, 5656 EINDHOVEN (NL)
(74) Representative: Philips Intellectual Property & Standards

(57) **Abstract**

The present invention relates to a device for generating a medical image of a region of interest (ROI) of a subject indicating contrast-enhanced regions. The device comprises an input (31) configured to obtain a spectral image data set of the ROI of the subject, the spectral image data set comprising two or more spectral images; a processing unit (32) and an output (33) configured to output a medical image of the ROI generated from the spectral image data set, in which the determined contrast-enhanced regions are indicated. The processing unit is configured to segment a plurality of anatomical sub-ROIs of the ROI in a virtual monoenergetic image and/or a full-energy image of the two or more spectral images; determine distribution information for at least one of the anatomical sub-ROIs in at least one contrast-sensitive image of the two or more spectral images having a higher contrast sensitivity and/or quantitative accuracy than the virtual monoenergetic image used for the segmentation, the distribution information indicating the tonal distribution of pixels of the respective anatomical sub-ROI; and determine contrast-enhanced regions of the at least one anatomical sub-ROI in the at least one contrast-sensitive image by determining a threshold based on the determined distribution information of the at least one anatomical sub-ROI and determining pixels of the at least one anatomical sub-ROI having pixel values above the determined threshold.

## Description

### FIELD OF THE INVENTION

The present invention relates to a device, a system and a method for generating a medical image of a region of interest (ROI) of a subject (e.g. a patient) indicating contrast-enhanced regions.

### BACKGROUND OF THE INVENTION

Delayed myocardial enhancement is a highly valuable imaging technique used in the diagnosis of a variety of diseases including myocardial viability, cardiomyopathy, myocarditis and other infiltrative myocardial processes. Late enhancement is mostly appraised at MR, but more recently also CT feasibility has been shown, where CT has a much wider availability and lower costs than MR.

More generally, imaging of contrast enhancements, preferably early enhancements in the early stage of the administration of a contrast agent and late enhancement in the late stage of the administration of a contrast agent, are of interest for the diagnosis in different medical applications and for different organs, such as myocardial diseases, heart diseases, liver perfusion analysis and brain stroke analysis.

### SUMMARY OF THE INVENTION

It is an object of the present invention to provide a device, a system and a method for

In a first aspect of the present invention a device for generating a medical image of a region of interest (ROI) of a subject indicating contrast-enhanced regions is presented, the device comprising:
- an input configured to obtain a spectral image data set of the ROI of the subject, the spectral image data set comprising two or more spectral images;
- a processing unit configured to
   - segment a plurality of anatomical sub-ROIs of the ROI in a virtual monoenergetic image and/or a full-energy image of the two or more spectral images;
   - determine distribution information for at least one of the anatomical sub-ROIs in at least one contrast-sensitive image of the two or more spectral images having a higher contrast sensitivity and/or quantitative accuracy than the virtual monoenergetic image used for the segmentation, the distribution information indicating the tonal distribution of pixels of the respective anatomical sub-ROI; and
   - determine contrast-enhanced regions of the at least one anatomical sub-ROI in the at least one contrast-sensitive image by determining a threshold based on the determined distribution information of the at least one anatomical sub-ROI and determining pixels of the at least one anatomical sub-ROI having pixel values above the determined threshold; and
- an output configured to output a medical image of the ROI generated from the spectral image data set, in which the determined contrast-enhanced regions are indicated.

In a further aspect of the present invention an imaging system is presented comprising:
- an imaging device configured to acquire a spectral image data set of a region of interest, ROI, of a subject, the spectral image data set comprising two or more spectral images;
- a device according to any one of the preceding claims for generating a medical image of a region of interest of a subject indicating contrast-enhanced regions based on the acquired spectral image data set; and
- a display unit configured to display the synthesized medical image

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to provide segmentations of contrast enhancement regions (in particular late enhancement regions) on spectral image reconstructions, by virtue of identifying anatomical sub-regions of interest (sub-ROIs, i.e. parts of the ROI) on virtual monoenergetic images (also called virtual mono-energy images and abbreviated as VMIs; optionally on a full-energy image instead or in addition), and to derive one or more case-specific thresholds on spectral maps of highest contrast sensitivity, i.e., on at least one contrast-sensitive image having higher contrast sensitivity and/or quantitative accuracy than the virtual monoenergetic image used for the segmentation. For instance, myocardial regions as well as several calibration regions (e.g., anatomical sub-ROIs including the myocardium, left ventricle, right ventricle, aorta, etc.) may be segmented on one or more virtual monoenergetic images.

In the one or more contrast-sensitive image distribution information is determined, which is sometimes referred to as dynamic distribution, which relates to intensity distribution (rather than spatial distribution) and indicates the tonal distribution of pixels of the respective anatomical sub-ROI.

The one or more determined case-specific thresholds (e.g. one threshold per anatomical sub-ROI) are then used to determine pixels of the respective anatomical sub-ROI having pixel values above the threshold so that in a final medical image of the ROI generated from the spectral image data set contrast-enhanced regions can be indicated and visualized for easy recognition by a user, e.g. a physician, to support a user in making a diagnosis.

As input a spectral image data set, e.g. a single spectral CT image, of the ROI of the subject is used, which generally comprises two or more spectral images, e.g. one or more base images, a monoE40KeV image (or other monoenergetic image), an iodine map, a Zeff image, etc. Spectral images are generally acquired simultaneously (when using dual layer detectors or photon counting detectors) or almost simultaneously (when using dual sources, where there is a very small time shift). ROIs can be copied from one spectral image to other spectral images. An anatomical sub-ROI should at least be segmented in one of the spectral images, but the spectral images might be different for different anatomical sub-ROIs. The segmentation of one anatomical sub-ROI may be sufficient since the area inside the sub-ROI and outside of the sub-ROI may be used as two separate sub-ROIs.

A virtual monoenergetic image or a conventional (full-energy) image is preferably used for segmenting the anatomically relevant regions, called anatomical sub-ROIs herein, yielding a compromise between anatomy and contrast agent. Such an image may generally be used as well for contrast uptake measurement, i.e., for determining distribution information indicating the tonal distribution of pixels of the respective anatomical sub-ROI. Preferably, however, a different spectral image (which is less suitable for the segmentation), such as an iodine density map or an electron density map is used for determining the distribution information.

For the segmentation, generally different methods or known algorithms may be applied and the processing unit may be configured to segment the one or more anatomical ROIs by use of one or more of a model-based segmentation method, a trained computer system, and a trained algorithm.

According to an embodiment the processing unit is configured to erode and/or allow a user to erode the plurality of segmented anatomical sub-ROIs. This allows to manually or automatically correct the contour of a sub-ROI, which further improves the accuracy of the segmentation and may ensure that only the desired anatomy, e.g. the desired organ (such as the myocardium) is visible in the segmented sub-ROI.

According to another embodiment the processing unit is configured to determine, as distribution information for an anatomical sub-ROI, a histogram and/or one or more histogram-derived values, in particular one or more of a median value, a mean value, and a mode value. Preferably, for each anatomical sub-ROI a histogram and one or more histogram-derived values are determined. In this context, the mode value shall be understood as the data value that occurs most often in a data set. For a symmetric histogram, the values of the mean, median, and mode are all the same and are all located at the center of the distribution, but in general they are not the same, and the median may be the most robust one.

According to another embodiment the processing unit is configured to determine the distribution information in at least one of a iodine density map, an electron density map, and an effective atomic number (Zeff) map. These images generally show a higher contrast sensitivity and/or quantitative accuracy than a monoenergetic image (such as a monoE40KeV or monoE60KeV image or a monoenergetic image for another energy), which represents a compromise showing both contrast agent and anatomy with sufficient accuracy. A virtual monoenergetic image or a conventional (full-energy) image may be more advantageous for segmenting the anatomically relevant parts and is hence used for segmentation according to the present invention.

There are multiple embodiments how the distribution information can be used in order to determine contrast-enhanced region(s). In an embodiment, the processing unit may be configured to determine, per anatomical sub-ROI, multiple thresholds relative to a median or mean or mode value of the pixel values of the anatomical sub-ROI, and to determine iso-contour regions of the anatomical sub-ROI using the multiple thresholds. The output may then output the iso-contour regions as contrast-enhanced regions.

Hereby, the processing unit may be configured to determine the threshold based on the median or mean or mode values of the anatomical sub-ROIs, in particular by a linear combination or non-linear regression of the median or mean or mode values.

In another embodiment, the processing unit is configured to determine the threshold(s) by use of a metric, in particular a histogram similarity metric. Preferably, a metric which can compare two histograms and compute a scalar value describing their mutual similarity is used. Hereby, the processing unit may be configured to minimize the metric between contrast-enhanced regions and residual regions of an anatomical sub-ROI and/or to maximize the metric between contrast-enhanced regions and blood pool regions. Two different objectives may thus be optimized individually or simultaneously in conjunction. For the first objective (to minimize the metric), a threshold may be varied (yielding a putative trial separation into two regions, like foreground/background, for each trial) until the similarity (as measured by the metric, in particular the histogram similarity metric) between the contrasted and the uncontrasted region becomes minimal (best distinguishable). For the second objective (to maximize the metric), a threshold may be varied until the similarity of the putative contrasted region becomes highest with regions that are definitely known to be filled with (pure) contrast (so called blood-pool regions like the cardiac ventricle or aorta).

In another embodiment the output is configured to output the medical image of the ROI in the form of a slice view and/or cylindrical projection view and/or atlas view and/or to indicate the determined contrast-enhanced regions by use of different colors or gray values.

The spectral image data set may generally be a spectral CT image data set or a photon counting CT image data set, which may depend on the kind of detector that has been used for acquiring the spectral image data set. Further, the spectral image data set may comprise two or more of a spectral base image, a monoE40KeV image, an iodine map image, and a Zeff image.

The proposed imaging system comprises, besides the device described above, an imaging device, e.g. a spectral CT scanner, and a display unit, such as a screen of a computer or workstation. In addition, a user interface, e.g. a touch screen, pointer, computer mouse, etc., may be provided that is configured to allow a user to provide user input to interactively mark the anatomical sub-ROIs in at least one of two or more spectral images of the spectral image data set.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention.
Fig. 2 shows a schematic diagram of an embodiment of a device according to the present invention.
Fig. 3 shows a schematic diagram of an embodiment of a method according to the present invention.
Fig. 4 showing an exemplary cardiac CT scan with semantic segmentation of anatomical sub-ROIs.
Fig. 5 shows an exemplary visualization of case-specific late enhancement regions relative to myocardium.

### DETAILED DESCRIPTION OF EMBODIMENTS

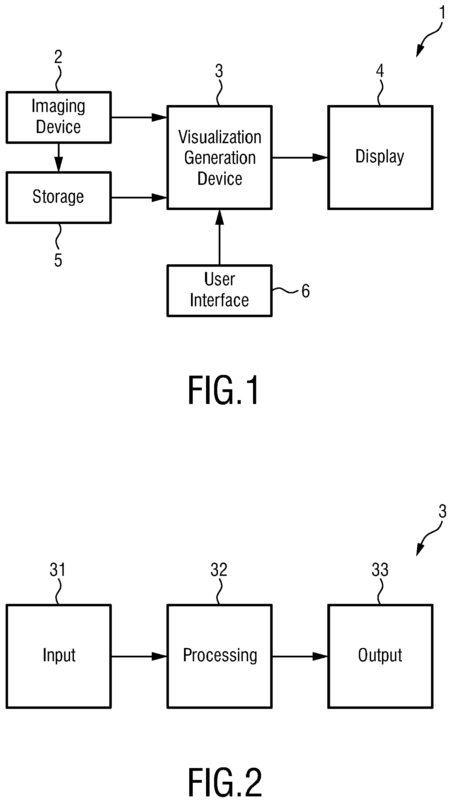

Fig. 1 shows a schematic diagram of an embodiment of a system 1 for generating a medical image of a region of interest (ROI) of a subject indicating contrast-enhanced regions according to the present invention. The system 1 comprises an imaging device 2 for acquiring a spectral image data set of the ROI of the subject, a device 3 for generating a medical image of the ROI of the subject indicating contrast-enhanced regions based on the acquired spectral image data set, and a display unit 4 for displaying the synthesized medical image.

The imaging device 2 is a CT device, in particular a spectral CT or photon-counting CT device, e.g. a conventionally used CT device for acquiring image data of a subject, e.g. of a certain body region of a patient. The imaging device acquires a spectral image data set comprising two or more spectral images. The spectral image data set may comprise one or more base images, a monoE40KeV image (or other monoenergetic image or another energy, e.g. 60KeV, 80KeV, etc.), a full-energy image, an iodine map, a Zeff image, etc., which may be acquired simultaneously or subsequently (almost simultaneously), depending on the implementation of the imaging device, in particular its detector and its radiation source.

The device 3 may e.g. be implemented as or in a processor or computer, in software and/or hardware. For instance, a programmed processor may be included in the device 3, which may execute a computer program that may be stored in a memory that is accessed by the processor.

The display unit 4 may e.g. be a monitor or screen of a PC, workstation, laptop tablet, etc., e.g. of a computer that represents the device 3 and is carrying out the method for generating a medical image of an ROI of a subject indicating contrast-enhanced regions according to the present invention.

The device 3 may directly obtain (i.e. retrieve or receive) the image data set directly from the imaging device 2 or from a storage 5, such as a buffer or memory or image repository, e.g. a patient record stored in a hospital's document management system, e.g. via a wired or wireless network.

A user interface 6, e.g. a touch screen, pointer, computer mouse, etc., may be provided so that a user can provide user input, for instance to interactively mark or correct the contour of the anatomical sub-ROIs in at least one of two or more spectral images of the spectral image data set.

Fig. 2 shows a schematic diagram of an embodiment of a device 3 for generating a medical image of the ROI of the subject indicating contrast-enhanced regions according to the present invention. The device may comprise circuitry, e.g. a processor, processing circuitry, a computer, dedicated hardware, etc. that carries out the functions of the device. In another embodiment, as shown in Fig. 2, separate units or elements may be used that together represent circuitry of the device 3.

In this embodiment the device 3 comprises an input unit 31 configured to a spectral image data set of the ROI of the subject, the spectral image data set comprising two or more spectral images. The spectral image data set may be obtained, e.g. from the imaging device 2 or the storage 5. For this purpose the input unit 31 may be directly or indirectly (e.g. via a network or bus) coupled or connected to the imaging device 2 or the storage 5. The input unit may thus e.g. be a (wired or wireless) communication interface or data interface, such as a Bluetooth interface, Wi-Fi interface, LAN interface, HDMI interface, direct cable connection, or any other suitable interface allowing data transfer to the device 2.

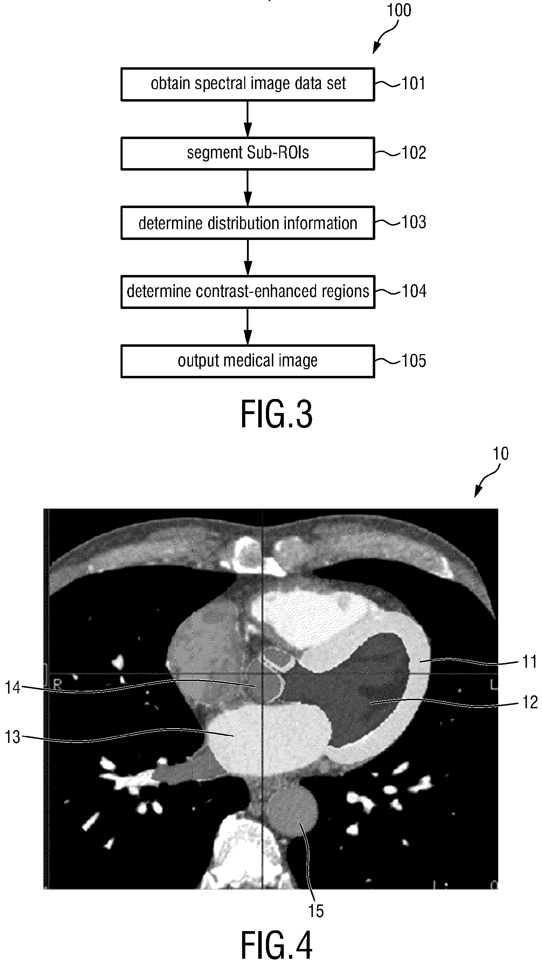

The device 3 further comprises a processing unit 32 configured to carry out the steps of the method according to the present invention as explained below with reference to Fig. 3. The processing unit 32 may be any kind of means configured to process the obtained spectral image data set and to generate a medical image of the ROI of the subject indicating contrast-enhanced regions there from. It may be implemented in software and/or hardware, e.g. as a programmed processor, computer, laptop, PC, workstation, etc.

The device 3 further comprises an output 33 configured to output a medical image of the ROI generated from the spectral image data set, in which the determined contrast-enhanced regions are indicated. The output 33 may generally be any interface that provides the generated visualization, e.g. transmits it to another device or provides it for retrieval by another device, e.g. transmits it to another computer or transfers it directly to the display 4 for displaying it. It may thus generally be any (wired or wireless) communication or data interface.

Fig. 3 shows a flow chart of a first embodiment of a method 100 for generating a medical image of the ROI of the subject indicating contrast-enhanced regions according to the present invention. The steps of the method may be carried out by the device 3, wherein the main steps of the method are carried out by the processing unit 32. The method may e.g. be implemented as computer program running on a computer or processor.

One of the aims of the idea implemented by the proposed method is the automated segmentation and quantification of contrast enhancement regions, using scan-specific thresholding, by virtue of different spectral maps (scan reconstructions) for the various building blocks (steps). In an exemplary, non-limiting application of the present invention, which will be referred to in the following explanation, late enhancement regions in the myocardium shall be segmented and quantified in order to visualize regions with enhanced contrast agent or reduce contrast agent indication potentially defective regions, e.g. regions with circulatory disorder.

In a first step 101, a spectral image data set of the ROI of the subject is obtained, the spectral image data set comprising two or more spectral images. The spectral image data set may be directly obtained from the imaging device, e.g. a spectral or photon-counting CT scanner, or from a storage, e.g. an image repository.

In a second step 102, a plurality of anatomical sub-ROIs of the ROI are segmented in a virtual monoenergetic image (VMI) of the two or more spectral images. Alternatively or additionally, a full-energy image may be used for the segmentation.

In the exemplary application, cardiac segmentation on a spectral VMI is performed, i.e., the cardiac region is automatically detected and segmented (delineated) into several anatomical sub-ROIs, e.g. myocardium, left ventricle, right ventricle, aorta, etc. This is illustrated in Fig. 4 showing a cardiac CT scan 10 with semantic segmentation of the following anatomical sub-ROIs: myocardium 11, left ventricle 12, right ventricle 13, ascending aorta 14, descending aorta 15. If necessary, sub-ROIs may be defined to improve robustness, e.g., by (manually or automatically) eroding the segmented regions.

The automatic segmentation can be based on, e.g., model-based segmentation (MBS) or other machine learning based (ML-based) semantic segmentations (e.g., deep CNNs). The segmentation, e.g. a segmentation algorithm, should rely (solely or at least mainly) on a VMI (e.g., VMI-40keV) having high contrast sensitivity, since, in a late enhancement image, most of the contrast agent has washed out already. The VMI should, however, still have enough conventional appearance such that standard segmentation models are applicable.

In a third step 103, distribution information for at least one of the anatomical sub-ROIs is determined in at least one contrast-sensitive image of the two or more spectral images having a higher contrast sensitivity and/or quantitative accuracy than the VMI used for the segmentation. The distribution information indicates the tonal distribution of pixels of the respective anatomical sub-ROI. Generally, the VMI (or full-energy image) used for the segmentation may be used in this step, but a different spectral image is preferred for this contrast uptake measurement.

In the exemplary application, regional histogram analysis may be performed on a contrast agent sensitive (CA-sensitive) image. Histograms and histogram-derived values (e.g., mean, median or mode values) are computed for all anatomical sub-ROIs, in particular the myocardium, left and right ventricle blood pools, aorta, etc. This should be performed on one or more spectral maps with highest contrast sensitivity and/or quantitative accuracy (e.g., an iodine density map, an electron density map, an effective atomic number (Zeff) map).

In a fourth step 104, contrast-enhanced regions of the at least one anatomical sub-ROI are determined in the at least one contrast-sensitive image by determining a threshold based on the determined distribution information of the at least one anatomical sub-ROI and determining pixels of the at least one anatomical sub-ROI having pixel values above the determined threshold. Different options are available for implementing this step to identify contrast-enhanced regions.

According to a first option a pseudo-color map or iso-contour regions are used, which may be scaled by median or mean or mode values. For instance, in the exemplary application, within the myocardial region, on the spectral map volume, iso-contour regions are computed using a series of thresholds relative to the median (or mean or mode) values of the anatomical sub-ROI, e.g., using the median (or mean or mode) value of the myocardium as zero level, and the median (or mean or mode) values of the blood-pool regions as maximum level. The resulting iso-contour regions are offered as contrast-enhanced candidate regions, e.g. as late contrast enhancement candidate regions, to the user (e.g. medical practitioner, physician, etc.) for visual appraisal or interactive choice.

According to a second option thresholding by optimized linear combination of median (or mean or mode) values is applied. A single scan-specific threshold (on the spectral map) is computed from a formula (e.g., a linear combination or non-linear regression) using the median (or mean or mode) values of the anatomical sub-ROIs. The (comparatively few) parameters have preferably been optimized on a certain number of earlier cases with manual ground truth. In the exemplary application, regions in the myocardium surpassing this scan-specific threshold are marked and quantified as contrast-enhanced regions, in particular late-enhancement regions.

According to a third option an automatic scan-specific threshold (e.g. for the myocardium) is applied, which may be derived automatically by an algorithm identifying a threshold that minimizes a histogram metric (e.g., mutual, information, entropy, Kullback-Leibler divergence, covariance, Otsu-distance, etc.) between the contrast-enhanced regions (e.g. myocardial late enhancement regions) and the residual regions of an anatomical sub-ROI (e.g. residual regions of the myocardium), and that at the same time or alternatively maximizes the histogram metric between the contrast-enhanced regions and blood pool regions. Regions in the anatomical sub-ROI (e.g. myocardium) surpassing this scan-specific threshold are marked and quantified as contrast-enhanced regions, e.g. late enhancement regions. In other words, different thresholds may be "tested", and for all pixels having pixel values below the respectively test threshold a histogram is prepared. The more diverse a histogram is, the better is the respective threshold. The best threshold is then finally used.

In a fifth step 105, a medical image of the ROI generated from the spectral image data set is output, in which the determined contrast-enhanced regions are indicated. For this visualization of the results various options exist as well. For instance, the results of each of the above options of the fourth step 104 can be represented in standard slice-wise view (or reformatted e.g., as short-axis or long-axis view), as well as in standardized cylindrical projection views ("bull's eye"). Moreover, all results can be summarized (e.g., means or medians or modes) per myocardial segment atlas (as defined by the American Heart Association, AHA).

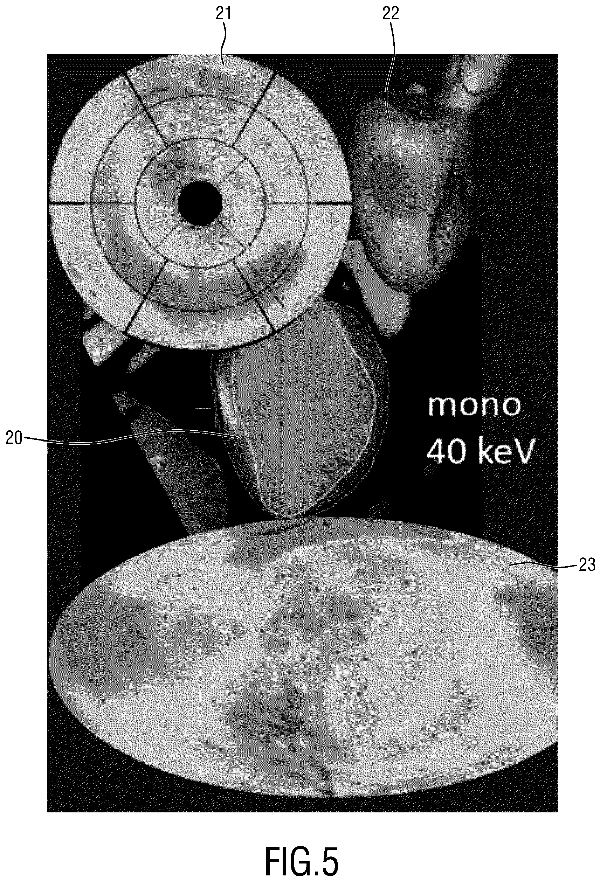

Fig. 5 shows an exemplary visualization of case-specific late enhancement regions relative to myocardium (scanned seven minutes after CA injection), using a color scale to signify spectral iodine density above versus below the median value of the myocardium, which is segmented automatically by MBS on the basis of a virtual mono-energy-40keV image, as shown by the delineations in the center of Fig. 5). Fig. 5 particularly shows the following representations: Standard HU values in slice-wise long-axis view 20 (in the center), a cylindrical projection 21 (bull's eye with AHA segments; top left), a 3D mesh view 22 (top right) and a flattened-map-projection 23 (bottom). Each of these views 20 to 23 or a combination of two or more of these views may represent a medical image of an ROI of a subject indicating contrast-enhanced regions as generated according to the present invention.

According to an embodiment, for all the above options, a prior three-dimensional smoothing (e.g., Gaussian or edge-preserving) of the chosen spectral map (not the anatomical sub-ROIs) is performed, so that the resulting contrast-enhanced regions exhibit finer or coarser granularity, depending on smoothing filter width.

The present invention provides one or more of the following advantages.

Conventional (non-spectral) CT imaging is an established standard, but has lower sensitivity to contrast agent in comparison to dual-energy, dual-layer, or photon-counting CT. Anatomical contours of the e.g. the myocardium, in particular the left cardiac ventricle, are not well visible on conventional CT, because in the late enhancement phase, the contrast agent has already washed out mostly. Such anatomical contours can be visualized by use of the present invention.

Contrast enhancement is manifesting optimally on spectral maps such as iodine density, electron density, effective atomic number, etc., however on these image types, the standard semantic segmentation models (e.g., MBS, Deep CNNs) may not be applied (or would have to be re-trained, at substantial costs). This is solved by the present invention by using a monoenergetic image for the initial segmentation. Further, as an analytical algorithm, no ML training data is required (with its accompanying annotation, sampling, imaging protocol coverage, and regulatory efforts). Still further, standard segmentation models developed on conventional CT data can be used, an no re-training of the semantic segmentation models on spectral CT images is required.

Experimental evidence shows that the use of fixed thresholds for significant enhancement is not satisfactory, as general contrast agent concentration depends on a number of imaging protocol specifics (contrast agent injection, amount and timing) and patient specific parameters (blood volume, circulation, weight, etc.). According to the present invention, optimal case-specific thresholds are determined per scan.

For a medical user, bringing together quantitative information from multiple three-dimensional spectral image volumes by unaided purely visual appraisal is cumbersome, time-consuming, and prone to error. The present invention overcomes this problem by automatic processing of the spectral images of the spectral image data set.

The present invention makes full use of the multiple spectral maps of a single scan, which are tedious to be comprehended side by side in a purely visual appraisal, and some of which are more useful for segmentation, others for thresholding, others for scaling, others for viewing, respectively.

"Enhancement" by contrast agent is a familiar radiological concept from conventional (non-spectral) imaging, so that thresholding as well as linear scaling relative to functionally meaningful cardiac compartments will be embraced with confidence and comprehension by the medical users (in comparison to "black-box" AI decision algorithms).

Compared to other cardiac applications that use multiple images like MRI or PET-CT, cardiac spectral CT differs, because all images have been acquired at identical times and additional registration steps to compensate for patient motion are not required. While the present invention has been described above with respect to cardiac diseases, it may also be applied to other organs such as, for instance, liver perfusion analysis or brain stroke analysis.

While the optimal spectral images are used for processing, the user might have the possibility to toggle between different spectral images/representations. Further, the user may toggle between different granularities/smoothness used for the contrast-agent sensitive spectral map before thresholding/scaling (choice of spatial filter width).

In summary, embodiments of the present invention provide for a combined usage of various spectral reconstruction image volumes: VMIs for semantic segmentation, and quantitative spectral maps thresholded by values derived relative to the median concentration values of myocardial and blood pool regions, resulting in a visual presentation of selectable conventional or spectral images.

The present invention may be applied to enable or facilitate a diagnosis in different medical applications and for different organs, such as myocardial diseases, heart diseases, liver perfusion analysis and brain stroke analysis.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (3) for generating a medical image of a region of interest, ROI, of a subject indicating contrast-enhanced regions, the device comprising:
- an input (31) configured to obtain a spectral image data set of the ROI of the subject, the spectral image data set comprising two or more spectral images;
- a processing unit (32) configured to
- segment a plurality of anatomical sub-ROIs of the ROI in a virtual monoenergetic image and/or a full-energy image of the two or more spectral images;
- determine distribution information for at least one of the anatomical sub-ROIs in at least one contrast-sensitive image of the two or more spectral images having a higher contrast sensitivity and/or quantitative accuracy than the virtual monoenergetic image used for the segmentation, the distribution information indicating the tonal distribution of pixels of the respective anatomical sub-ROI; and
- determine contrast-enhanced regions of the at least one anatomical sub-ROI in the at least one contrast-sensitive image by determining a threshold based on the determined distribution information of the at least one anatomical sub-ROI and determining pixels of the at least one anatomical sub-ROI having pixel values above the determined threshold; and
- an output (33) configured to output a medical image of the ROI generated from the spectral image data set, in which the determined contrast-enhanced regions are indicated.

2. Device according to claim 1,
wherein the processing unit (32) is configured to segment the one or more anatomical ROIs by use of one or more of a model-based segmentation method, a trained computer system, a trained algorithm.

3. Device according to any one of the preceding claims,
wherein the processing unit (32) is configured to erode and/or allow a user to erode the plurality of segmented anatomical sub-ROIs.

4. Device according to any one of the preceding claims,
wherein the processing unit (32) is configured to determine, as distribution information for an anatomical sub-ROI, a histogram and/or one or more histogram-derived values, in particular one or more of a median value, a mean value, and a mode value.

5. Device according to any one of the preceding claims,
wherein the processing unit (32) is configured to determine the distribution information in at least one of a iodine density map, an electron density map, and an effective-atomic-number map.

6. Device according to any one of the preceding claims,
wherein the processing unit (32) is configured to determine, per anatomical sub-ROI, multiple threshold relative to a median or mean or mode value of the pixel values of the anatomical sub-ROI, and to determine iso-contour regions of the anatomical sub-ROI using the multiple thresholds, and
wherein the output (33) is configured to output the iso-contour regions as contrast-enhanced regions.

7. Device according to any one of claims 1 to 5,
wherein the processing unit (32) is configured to determine the threshold based on the median or mean or mode values of the anatomical sub-ROIs, in particular by a linear combination or non-linear regression of the median or mean or mode values.

8. Device according to any one of claims 1 to 5,
wherein the processing unit (32) is configured to determine the threshold by use of a metric, in particular a histogram similarity metric.

9. Device according to claim 8,
wherein the processing unit (32) is configured to minimize the metric between contrast-enhanced regions and residual regions of an anatomical sub-ROI and/or to maximize the metric between contrast-enhanced regions and blood pool regions.

10. Device according to any one of the preceding claims,
wherein the output (33) is configured to output the medical image of the ROI in the form of a slice view and/or cylindrical projection view and/or atlas view and/or to indicate the determined contrast-enhanced regions by use of different colors or gray values.

11. Device according to any one of the preceding claims,
wherein the spectral image data set is a spectral CT image data set or a photon counting CT image data set or an MRI image data set or a PET-CT image data set, and/or comprises two or more of a spectral base image, a monoE40KeV image, an iodine map image, and a Zeff image.

12. Imaging system (1) comprising:
- an imaging device (2) configured to acquire a spectral image data set of a region of interest, ROI, of a subject, the spectral image data set comprising two or more spectral images;
- a device (3) according to any one of the preceding claims for generating a medical image of a region of interest, ROI, of a subject indicating contrast-enhanced regions based on the acquired spectral image data set; and
- a display unit (4) configured to display the synthesized medical image.

13. Imaging system according to claim 12,
further comprising a user interface (6) configured to allow a user to provide user input to interactively mark the anatomical sub-ROIs in at least one of two or more spectral images of the spectral image data set.

14. Method for generating a medical image of a region of interest, ROI, of a subject indicating contrast-enhanced regions, the method comprising:
- obtaining a spectral image data set of the ROI of the subject, the spectral image data set comprising two or more spectral images;
- segmenting a plurality of anatomical sub-ROIs of the ROI in a virtual monoenergetic image and/or a full-energy image of the two or more spectral images;
- determining distribution information for at least one of the anatomical sub-ROIs in at least one contrast-sensitive image of the two or more spectral images having a higher contrast sensitivity and/or quantitative accuracy than the virtual monoenergetic image used for the segmentation, the distribution information indicating the tonal distribution of pixels of the respective anatomical sub-ROI;
- determining contrast-enhanced regions of the at least one anatomical sub-ROI in the at least one contrast-sensitive image by determining a threshold based on the determined distribution information of the at least one anatomical sub-ROI and determining pixels of the at least one anatomical sub-ROI having pixel values above the determined threshold; and
- outputting a medical image of the ROI generated from the spectral image data set, in which the determined contrast-enhanced regions are indicated.

15. Computer program comprising program code means for causing a computer to carry out the steps of the method as claimed in claim 14 when said computer program is carried out on the computer.
